# EUROPEAN PATENT SPECIFICATION

(11) **EP 1 310 213 B1**
(45) Date of publication and mention of the grant of the patent: **14.09.2005**
(21) Application number: 02425679.4
(22) Date of filing: 07.11.2002
(51) Int. Cl.: A61B 5/11, A63B 26/00

(54) **Apparatus for assessing the automatic reflex function, to be used, in particular, for evoking the antigravity reflex**
Gerät zur Ermittlung von Reflexen ausgelöst durch freien Fall
Appareil pour évaluation de reflexes crées par chute libre

(30) Priority: 12.11.2001 IT FI20010213
(43) Date of publication of application: 14.05.2003
(73) Proprietor: Polymed S.r.l., 50020 Sambuca (Firenze) (IT); Betti, Giovanni, 50122 Firenze (IT); Piga, Roberto, Gaiole in Chianti (Siena) (IT)
(72) Inventor: Betti, Giovanni, 50122 Firenze (IT); Piga, Roberto, Gaiole in Chianti (Siena) (IT)
(74) Representative: Barberi, Vittorio

(56) References cited:
- EP-A- 0 519 836
- EP-A- 0 997 723
- DE-U- 29 600 427

## Description

The present invention refers to an apparatus for assessing the automatic reflex function to be used, in particular, for evoking the antigravity reflex.

It is known that all the living beings possess defence and life-preserving mechanisms. Among the human beings, these mechanisms make use of the nervous system which allows the activation of defences in short periods of time. Automatic reflexes is the expression mentioned for indicating when the response to a "disturbance" takes place, initially, without any involvement of the Central Nervous System (CNS).
A typical example is that when a hot surface is inadvertently touched with a hand and the latter is retracted immediately before the effect is sensed by the CNS. The situation described by the example is likely to occur in the everyday life, whereas the activation of other reflexes is more unusual. Among the latter, there is the so-called "antigravity reflex" that can be experienced both within an airplane when coming across an air pocket, and upon walking, when the support of the ground is abruptly lost, which is of particular importance in the motor activity. When the antigravity reflex is activated, all the muscles of the body, which operate phisiologically under a gravity regime, begin working automatically. Persons having suffered from traumas or cortical and spinal marrow pathologies exhibit an alteration of the functionality of such reflex.

EP-A- 0 997 723 discloses a drop testing that is performed by controlling the position of a product to be tested with respect to the drop surface until just before the initial impact and then the product is allowed to impact like a free body. EP-A-0 519 836 relates to an apparatus for detecting and correcting balance anomalies of a patient, of the type comprising a first lower plate which is fixed with respect to the ground, and a second, moving plate held with respect to the first by elastic deformable means, on which a patient stands, first means for displaying the horizontality of the moving plate, of the air or spirit level type, and second display means.

The object of the present invention is to provide an instrument which allows assessing, in a systematic and repeatable manner, the functionality of the reflex. This result has been achieved, according to the invention, by adopting the idea of making an apparatus having the characteristics indicated in claim 1. Further characteristics being set forth in the dependent claims.

The advantages of the present invention lie essentially in that it is possible to evaluate the antigravity reflex during a rehabilitation stage, in order to check a patient's progress and to attest his/her recovery degree, for example, in the medical and insurance field, on the basis of substantially objective parameters. Moreover, in the sport field it has been found experimentally that when using the apparatus for testing traumatized athlets, the same apparatus performs also a training function. The modern training techniques actually provide for the selective improvement of muscles involved in a specific athletic performance.

The present instrument which evokes, under an operator-controlled situation, a sudden antigravity reflex, makes it possible above all, but not exclusively, the activation of the antigravity muscular system which is represented, in the legs, by the muscles of extensor system, femoral quadriceps, gastrocnemius and plantar flexors. It is thus possible to exalt the antigravity reaction of the muscles being used, for example, in the jump and race and, at the same time, to simulate and treat the lack of coordination, which follows a sudden and reflexed action, in the upper limbs. Again in the sport field, but not exclusively, the present instrument can be used to assess the fatigue and stress condition, as the gravity reflex is affected by these factors. Finally, the apparatus of the present invention is highly resistant and maintains substantially unchanged its characteristics even after prolonged periods of use, by requiring and extremely limited maintenance.

These and other advantages and characteristics of the invention will be best understood from a reading of the following description in conjunction with the attached drawings given as a practical exemplification of the invention, but not to be considered in a limitative sense, wherein:
- Fig. 1 is a schematic plan view of a possible embodiment of the apparatus according to the present invention;
- Fig. 2 is a view in section taken on line II-II in Fig. 1 of the apparatus shown in Fig. 1, with parts taken away to highlight others;
- Fig. 3 is a schematic plan view of a further possible embodiment of the apparatus.

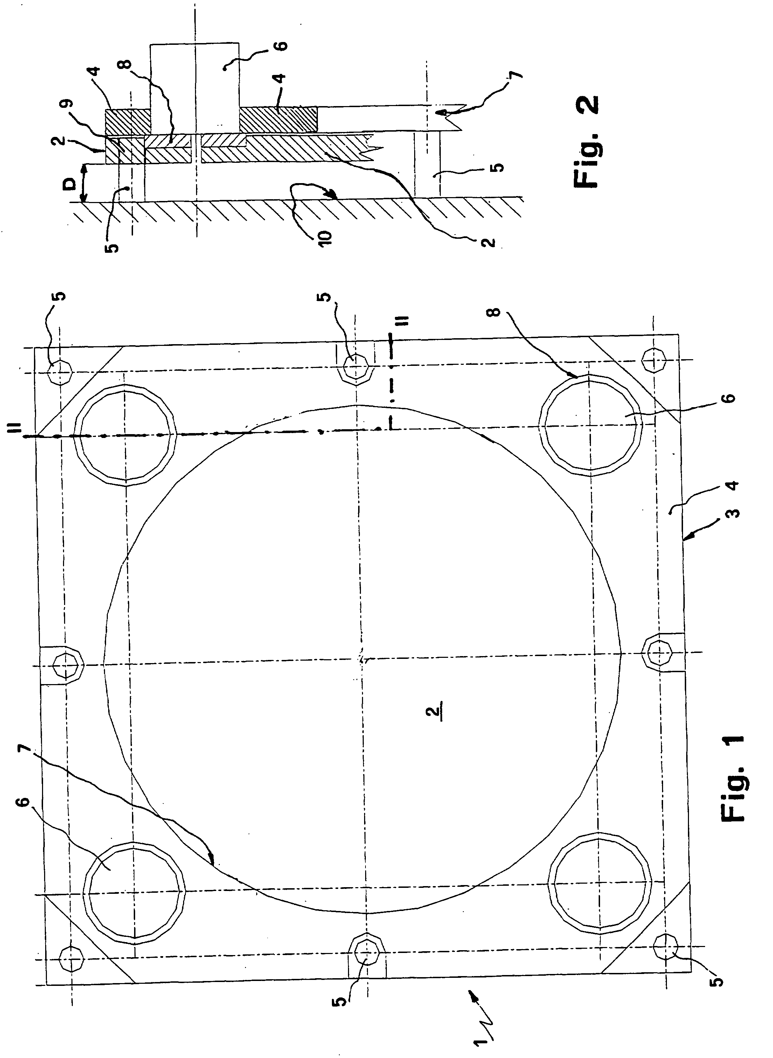

With reference to the figures of the attached drawings, an apparatus according to the invention comprises a platform 2 and a relevant support structure 3, connected to each other by removable means 6, 8.
The support structure shown by way of example in the drawings of Figs. 1 and 2 consists of a horizontal plate 4 substantially square in plan view, with a central hollow and circular development region 7 and provided with support feet 5 resting on the ground or other suitable abutment surface. Provided on the support structure 3, and solid to the fixed horizontal plate 4, are blocks of electromagnets 6 (in number of four in the illustrated example) connected with relevant actuators of conventional type and, therefore, being omitted in the drawings. The actuators of the electromagnets 6 can be connected to suitable data-processing means (for example, an electronic board) so as to operate the apparatus, according to a preset programs of diagnosis, possibly interfaced to measuring devices of various type.
The platform 2 (which in Fig. 2 has some parts taken away for the sake of clarity) is shaped substantially like a square, similarly to plate 4 of the support structure 3 and is disposed parallel to the same plate 4. The platform, moreover, has a plurality of holes 9 disposed in correspondence of the feet 5 of structure 3. The diameter of the holes 9 is such as to allow a free sliding of the platform 2 along the stem of feet 5, that is, a free vertical displacement thereof with very much reduced friction of platform 2 relative to structure 3, with a vertical guide defined by the stems of feet 5.
Provided on the platform are four pads of soft iron, correspondingly located with respect to the electromagnets 6 so as to form resolvable matchable means for association with the platform 2 and structure 3. It is understood that, alternatively, the parts in ferromagnetic material may be provided onto the support structure 3, with the electromagnets being solid to the platform 4.

On use, a person is made to stand on the platform 2, in correspondence of the central region thereof, that is, the region having circular development and being accessible through the opening 7 of structure 3.
In the stand-by configuration, the blocking means (electromagnets 6) are activated and, thus, the platform is in the upper position.
At the bottom, the abutment surface may be the ground or the floor 10 or other surface, possibly coated with a layer of shock-absorbing material, for example sponge rubber.
To check the reactions to the antigravity reflex of a person under test, the electromagnets 6 are deactivated and, accordingly, the platform 2 moves abruptly downwards.
The vertical stroke D performed by the platform 2 is relatively short; its extension may be in the range of 5 to 500 mm; good results have been found experimentally with a value of about 30 mm.

The time during which the platform is released is extremely short, betwee 1 msec and 1 sec; good results have been obtained experimentally with a time of about 20 msec. It is understood that the release time (which is relatively short) may be preceded by a preparation step of variable length; in practice, what is important in view of the reactions assessment is the time of the actual release.

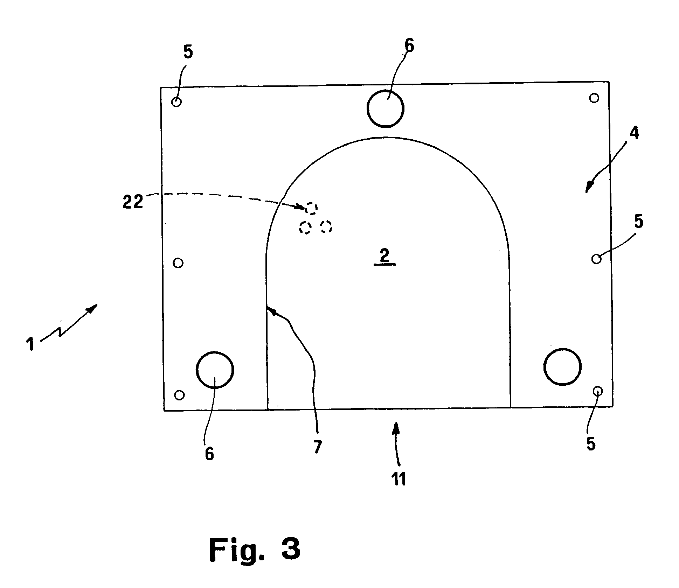

Shown in Fig. 3 is a further embodiment of the support structure 1. It is made up of a horizontal plate 4 substantially rectangular in plan view and provided with bearing feet 5. In this example, the plate 4 exhibits a hollow region 7 open on the side 11, with a peripheral edge of substantially "U" shape, and actually developing so that a side of the structure, formed by the plate 4 and platform 2, is left open. This configuration is able to offer a major comfort in use as it limits the possibility of interference by the person under test.

The operation is similar to what has been described above, and like parts are designated by the same numerals. The schematic plan view shows the electromagnets 6, but not the corresponding pads 8 though provided.

Although it has been found experimentally that the coupling of electromagnetic type is resulted particularly efficient, it is not exluded the possibility of other embodiments of the resolvable matchable means for association with platform 2 and structure 3. For example, provision may be made for a plurality of tongues (or rods) retractable under control (in number of three, for example) disposed and acting between the platform and the structure, and suitable for sustaining, when inserted into corresponding seats, the platform and releasing it upon a relevant activation command. It is understood that the driving of the tongues must be properly synchronized to attain a substantially simultaneous desengagement thereof.
Moreover, the platform may exhibit one or more holes 22 able to reduce the effect of the resistance offered by the air upon the drop of platform 2 (in conjunction with the relevant load of the user standing thereon) once it is released. Fig. 3 shows only three such holes represented with dotted line.

## Claims

1. Apparatus (1) for assessing the automatic reflex function, to be used, in particular, for evoking the antigravity reflex, said apparatus (1) comprising a platform (2), intended for sustaining a person under control, a relevant support structure (3) with respect to which said platform (2) is movable vertically through a stroke (D) of preset and limited extent, apparatus (1) **characterized in that** it comprises resolvable matchable means (6, 8) of magnetic type for attaching the structure (2) to the platform (3) and able to release the platform within a short time by making it to pass abruptly from the upper end of said stroke, where it is in stand-by position, to the lower end at the bottom or lower stop position.

2. Apparatus according to claim 1, **characterized in that** said platform (2) is made at least in part from a ferromagnetic material or it is solid to parts made from such material, and **in that** said removable association means comprise one or more electromagnets (6) disposed and acting between said fixed structure (3) and said platform (2).

3. Apparatus according to claim 1, **characterized in that** said support structure (3) is made at least in part from a ferromagnetic material or it is solid to parts made from such material, and **in that** said removable association means comprise one or more electromagnets (6) disposed and acting between said platform (2) and said fixed structure (3).

4. Apparatus according to claim 2 or 3, **characterized in that** said parts in ferromagnetic material are made up of pads (8) in soft iron.

5. Apparatus according to claim 4, **characterized in that** said pads (8) are disposed in peripheral regions of said platform (2).

6. Apparatus according to claim 4, **characterized in that** said pads (8) are disposed in peripheral regions of said structure (3).

7. Apparatus according to claim 1, **characterized in that** said preset vertical stroke (D) is in the range of 5 to 500 mm.

8. Apparatus according to claim 1, **characterized in that** said preset vertical stroke (D) is about 30 mm.

9. Apparatus according to claim 1, **characterized in that** said support structure (3) is provided with a bearing base or feet (5) which maintain the said platform (2) raised from an abutment surface (10) by an extent ranging from 5 to 500 mm.

10. Apparatus according to claim 1, **characterized in that** said support structure (3) is provided with a bearing base or feet (5) which maintain the said platform (2) raised from an abutment surface (10) by an extent of about 30 mm.

11. Apparatus according to claim 1, **characterized in that** said platform (2) exhibits a plurality of through holes (9), said bearing structure (3) being provided with a corresponding plurality of bearing feet (5) inserted into said holes (9) and able to define a vertical guide along said stroke of platform (2).

12. Apparatus according to claim 1, **characterized in that** said short time is between 1 msec and 1 sec.

13. Apparatus according to claim 1, **characterized in that** said short time is of about 20 msec.

14. Apparatus according to claim 1, **characterized in that** said platform (2) has a plurality of through holes (22) able to reduce the effect of the resistance offered by the air during said vertical stroke of platform (2).

15. Apparatus according to claim 1, **characterized in that** said support structure (3) consists of a horizontal plate (4) having a hollow region (7) through which said platform (2) is accessible from above.

16. Apparatus according to claim 15, **characterized in that** said hollow region (7) is substantially of "U" shape.

17. Apparatus according to claim 15, **characterized in that** said hollow region (7) is substantially of circular shape.

## Patentansprüche

1. Vorrichtung (1) zur Bewertung der automatischen Reflexfunktion, die insbesondere verwendet wird, um den Antigravitationsreflex hervorzurufen, wobei diese Vorrichtung (1) eine Plattform (2), die dazu dient, eine zu untersuchende Person zu tragen. und eine entsprechende Tragstruktur (3) umfasst, bezüglich derer die Plattform (2) in senkrechter Richtung über eine Hubweite (D) mit vorgegebener und begrenzter Größe bewegbar ist, wobei die Vorrichtung (1) **dadurch gekennzeichnet ist, dass** sie lösbare, anpassbare Magneteinrichtungen (6, 8) zum Befestigen der Struktur (2) an der Plattform (3) umfasst, die in der Lage sind, die Plattform in einem kurzen Zeitraum **dadurch** freizugeben, dass sie sie veranlassen, sich abrupt vom oberen Ende ihrer Hubweite, an dem sie sich in ihrer Stand-By-Position befindet, zum unteren Ende am Boden bzw. der unteren Stop-Position zu bewegen.

2. Vorrichtung nach Anspruch 1, **dadurch gekennzeichnet, dass** die Plattform (2) zumindest teilweise aus einem ferromagnetischen Material hergestellt oder mit Teilen fest verbunden ist, die aus einem solchen Material bestehen, und dass die lösbaren Zuordnungseinrichtungen einen oder mehrere Elektromagnete (6) umfassen, die zwischen der festen Struktur (3) und der Plattform (2) angeordnet sind und zwischen diesen beiden Einheiten wirken.

3. Vorrichtung nach Anspruch 1, **dadurch gekennzeichnet, dass** die Tragstruktur (3) zumindest teilweise aus einem ferromagnetischen Material hergestellt oder fest mit Teilen verbunden ist, die aus einem solchen Material bestehen, und dass die lösbaren Zuordnungseinrichtungen einen oder mehrere Elektromagnete (6) umfassen, die zwischen der Plattform (2) und der festen Struktur (3) angeordnet sind und zwischen diesen Einheiten wirken.

4. Vorrichtung nach Anspruch 2 oder 3, **dadurch gekennzeichnet, dass** die Teile aus ferromagnetischem Material aus Platten (8) aus Weicheisen bestehen.

5. Vorrichtung nach Anspruch 4, **dadurch gekennzeichnet, dass** die Platten (8) in Umfangsbereichen der Plattform (2) angeordnet sind.

6. Vorrichtung nach Anspruch 4, **dadurch gekennzeichnet, dass** die Platten (B) in Umfangsbereichen der Struktur (3) angeordnet sind.

7. Vorrichtung nach Anspruch 1. **dadurch gekennzeichnet, dass** der voreingestellte vertikale Hub (D) im Bereich von 5 bis 500 mm liegt.

8. Vorrichtung nach Anspruch 1, **dadurch gekennzeichnet, dass** der voreingestellte vertikale Hub (D) ungefähr 30 mm beträgt.

9. Vorrichtung nach Anspruch 1, **dadurch gekennzeichnet, dass** die Tragstruktur (3) mit einer Tragbasis oder Füßen (5) versehen ist, welche die Plattform (2) so halten, dass sie über eine Anlageoberfläche (10) um 5 bis 500 mm angehoben ist.

10. Vorrichtung nach Anspruch 1, **dadurch gekennzeichnet, dass** die Tragstruktur (3) mit einer Tragbasis oder Füßen (5) versehen ist, welche die Plattform (2) um ungefähr 30 mm über einer Anlageoberfläche (10) angehoben halten.

11. Vorrichtung nach Anspruch 1, **dadurch gekennzeichnet, dass** die Plattform (2) eine Vielzahl von durchgehenden Löchern (9) aufweist, wobei die Tragstruktur (3) mit einer entsprechenden Vielzahl von Tragfüßen (5) versehen ist, die in diese Löcher (9) eingesetzt und in der Lage sind, eine vertikale Führung längs des Hubes der Plattform (2) zu bilden.

12. Vorrichtung nach Anspruch 1, **dadurch gekennzeichnet dass** die kurze Zeitspanne zwischen 1 ms und 1 s liegt.

13. Vorrichtung nach Anspruch 1, **dadurch gekennzeichnet, dass** die kurze Zeitspanne ungefähr 20 ms beträgt.

14. Vorrichtung nach Anspruch 1, **dadurch gekennzeichnet, dass** die Plattform (2) eine Vielzahl von durchgehenden Löchern (22) besitzt, die den Widerstandseffekt vermindern können, der durch die Luft während der vertikalen Hubbewegung der Plattform (2) bewirkt wird.

15. Vorrichtung nach Anspruch 1, **dadurch gekennzeichnet, dass** die Tragstruktur (3) aus einer hortzontalen Platte (4) besteht, die einen hohlen Bereich (7) aufweist, durch welchen die Plattform (2) von oben her zugänglich ist.

16. Vorrichtung nach Anspruch 15, **dadurch gekennzeichnet, dass** der hohle Bereich (7) im Wesentlichen U-förmig ist.

17. Vorrichtung nach Anspruch 15, **dadurch gekennzeichnet, dass** der hohle Bereich (7) im Wesentlichen kreisförmig ist.

## Revendications

1. Appareillage (1) pour l'évaluation du caractère functionnel de réflexes automatiques utilisables, en particulier, pour l'évocation du réflexe antigravité, ledit appareillage (1) comprenant une plate-forme (2), destinée à l'appui du sujet qu'il faut contrôler, une relative structure (3) de support, par rapport à laquelle ladite plate-forme (2) est mobile selon une course verticale (D) prédéterminée d'une ampleur contenue, appareillage (1) **caractérisé en ce que** comprend des moyens résolubles d'ablocage (6, 8) de type magnétique de la plate-forme (2) à la structure (3), aptes à relâcher la plate-forme en temps bref, en la faisant passer soudainement, de l'extrémité supérieure de ladite course en position de retenue à repos, à l'extrémité inférieure ou de fin course.

2. Appareillage selon la revendication 1, **caractérisé en ce que** ladite plate-forme (2) est réalisée au moins en partie en matériel ferromagnétique ou est solidaire à des parties en tel matériel, et que lesdits moyens résolubles comprennent un ou plusieurs électro-aimants (6) disposés et agissant entre ladite structure fixe (3) et ladite plate-forme (2).

3. Appareillage selon la revendication 1, **caractérisé en ce que** ladite structure de support (3) est réalisée au moins en partie en matériel ferromagnétique ou est solidaire à des parties de tel matériel, et que lesdits moyens résolubles d'association comprennent un ou plusieurs électro-aimants (6) disposés et agissant entre ladite plate-forme (2) et ladite structure fixe (3).

4. Appareillage selon les revendications 2 ou 3, **caractérisé en ce que** lesdites parties en matériel ferromagnétique sont constituées par des tampons (8) en fer doux.

5. Appareillage selon la revendication 4, **caractérisé en ce que** lesdits tampons (8) sont disposés sur des zones périphériques de ladite plate-forme (2).

6. Appareillage selon la revendication 4, **caractérisé en ce que** lesdits tampons (8) sont disposés sur des zones périphériques de ladite structure (3).

7. Appareillage selon la revendication 1, **caractérisé en ce que** ladite course verticale prédéterminée (D) est d'une valeur comprise entre 5 et 500 mm.

8. Appareillage selon la revendication 1, **caractérisé en ce que** ladite course verticale prédéterminée (D) est de presque 30mm.

9. Appareillage selon la revendication 1, **caractérisé en ce que** ladite structure de support (3) est pourvue d'une base ou de pieds de soutien (5) qui maintiennent ladite plate-forme (2) soulevée d'une superficie de butée (10) d'une valeur comprise entre 5 et 500 mm.

10. Appareillage selon la revendication 1, **caractérisé en ce que** ladite structure de support (3) est pourvue d'une base ou de pieds de soutien (5) qui maintiennent ladite plate-forme (2) soulevée d'une superficie de butée (10) d'une valeur de presque 30mm.

11. Appareillage selon la revendication 1, **caractérisé en ce que** ladite plate-forme (2) présente une pluralité de trous passants (9), ladite structure de support (3) étant pourvue d'une correspondante pluralité de pieds de soutien (5) Insérés dans lesdits trous (9) et qui sont aptes à définir une guide verticale le long de ladite course de la plate-forme (2).

12. Appareillage selon la revendication 1, **caractérisé en ce que** ledit temps bref est d'une valeur comprise entre 1 msec et 1 sec.

13. Appareillage selon la revendication 1, **caractérisé en ce que** ledit temps bref est de presque 20 msec.

14. Appareillage selon la revendication 1, **caractérisé en ce que** ladite plate-forme (2) présente une pluralité de trous passants (22), aptes à diminuer l'influence de la résistance offerte par l'air pendant ladite course verticale de la plate-forme (2).

15. Appareillage selon la revendication 1, **caractérisé en ce que** ladite structure de support (3) se compose d'une plaque horizontale (4) qui présente une zone creuse (7) à travers laquelle est accessible du haut de ladite plate forme (2).

16. Appareillage selon la revendication 15, **caractérisé en ce que** ladite zone creuse (7) est conformée essentiellement en « U ».

17. Appareillage selon la revendication 15, **caractérisé en ce que** ladite zone creuse (7) a une forme essentiellement circulaire.
